# NEUE EUROPÄISCHE PATENTSCHRIFT

(11) **EP 1 200 362 B2**
(45) Veröffentlichungstag und Bekanntmachung des Hinweises auf die Entscheidung über den Einspruch: **11.10.2006**
(45) Hinweis auf die Patenterteilung: 14.05.2003
(21) Anmeldenummer: 00954440.4
(22) Anmeldetag: 06.07.2000
(51) Int. Cl.: C02F 3/28

(54) **VERFAHREN UND VORRICHTUNG ZUM REINIGEN VON ABWASSER**
METHOD AND DEVICE FOR THE PURIFICATION OF WASTEWATERS
PROCEDE ET DISPOSITIF D'EPURATION D'EAUX USEES

(30) Priorität: 06.07.1999 DE 19931085
(43) Veröffentlichungstag der Anmeldung: 02.05.2002
(73) Patentinhaber: OTV SA, 94417 Saint-Maurice Cédex (FR)
(72) Erfinder: ROSSMANITH, Peter, 78239 Rielasingen-Worblingen (DE)
(74) Vertreter: Hilgers, Hans Hubert
(86) Internationale Anmeldenummer: PCT/EP2000/006413
(87) Internationale Veröffentlichungsnummer: WO 2001/002309

(56) Entgegenhaltungen:
- EP-A- 0 342 722
- DE-T- 69 703 899
- US-A- 4 609 460
- US-A- 5 518 618
- US-A- 5 565 098
- PATENT ABSTRACTS OF JAPAN vol. 010, no. 090 (C-337), 8. April 1986 (1986-04-08) & JP 60 220194 A (KURITA KOGYO KK), 2. November 1985 (1985-11-02)

## Beschreibung

Die Erfindung bezieht sich auf ein Verfahren zum Reinigen von Abwasser der im Oberbegriff von Anspruch 1 erläuterten Art.

Ein derartiges Verfahren und eine derartige Vorrichtung ist aus der EP 0 170 332 B1 bekannt. Die bekannte Vorrichtung arbeitet anaerob, bevorzugt nach dem bekannten UASB-Verfahren (Upflow-Anaerob-Sludge-Blanket), bei dem ein Behälter verwendet wird, in dessen unteren Bereich das zu reinigende Abwasser geleitet und aus dessen oberen Bereich das gereinigte Abwasser abgeleitet wird. Im Behälter sind anaerobe Mikroorganismen tätig. Zwischen dem Abwassereinlaß und dem Überlauf für das gereinigte Abwasser befinden sich im Behälter übereinandergestapelt angeordnete Gassammler in Form von Hauben, deren oberer Bereich über eine Leitung mit einer Gas-Schlamm-Trenneinrichtung verbunden ist. Durch die Tätigkeit der Mikroorganismen wird Gas erzeugt, das sich am Schlamm anlagert, so daß dieser als sogenannter Schwimmschlamm nach oben aufschwimmt. Dieser Schwimmschlamm wird durch die Haube eingefangen und gibt nach und nach sein Gas wieder ab, so daß er wieder schwerer wird und als sogenannter Sinkschlamm zurück auf den Boden sinkt. Das von den Pellets abgegebene Gas steigt zusammen mit den von den Hauben eingefangenen, freien Gasblasen weiter in den Leitungen nach oben und reißt dabei Schwimmschlammpartikel und Flüssigkeit mit, die in derGas-Schlamm-Trennkammer abgetrennt werden. Das Gas wird zweckmäßigerweise abgeführt, während die mitgerissene Flüssigkeit, die auch Schlammpartikel enthalten kann, in eine Falleitung gelangt, die zurück auf den Boden des Behälters führt. Das gereinigte Abwasser gelangt im oberen Bereich des Behälters über einen Überlauf in eine Ablaufleitung und wird abgezogen. Es hat sich jedoch herausgestellt, daß die Reinigungsleistung derartiger Behälter noch verbesserungsbedürftig ist.

Der Erfindung liegt somit die Aufgabe zugrunde, ein Verfahren und eine Vorrichtung zum Reinigen von Abwasser bereitzustellen, mit der auf konstruktiv einfache Weise die Reinigungswirkung optimiert wird.

Die Aufgabe wird durch die im Anspruch 1 angegebenen Merkmale gelöst. Durch das erfindungsgemäße Verfahren, Medium aus dem oberen Bereich des Behälters nochmals anzusaugen und wiederum in den Reinigungskreislauf zu bringen, wird ein Umlauf des Mediums im Behälter in Gang gesetzt oder verstärkt, was zu einer wesentlich verbesserten Reinigungswirkung führt, wobei durch die Ausnutzung des Gashebeeffekts des bereits abgetrennten Gases diese verbesserte Reinigungswirkung weder einen zusätzlichen Energieeinsatz noch komplizierte konstruktive Umbauten beispielsweise in bereits bestehenden Behältern erfordern.

Zweckmäßigerweise wird das angesaugte Medium zusammen mit denjenigen Inhaltsstoffen des Mediums, die bei der Gassammlung mitgerissen wurden, wieder zurück in den Behälter geleitet.

Anspruch 3 beschreibt eine besonders bevorzugte Vorrichtung zum Durchführen des erfindungsgemäßen Verfahrens.

Erfindungsgemäß ist zum Verwirklichen der verbesserten Reinigungswirkung lediglich eine zusätzliche Leitung zwischen dem oberen Teil des Behälters und der Gassammelleitung vorzusehen, die ohne weiteres auch in bereits bestehende Behälter nachgerüstet werden kann.

Vorteilhafte Weiterbildungen der erfindungsgemäßen Vorrichtung sind den Unteransprüchen 4 bis 10 zu entnehmen.

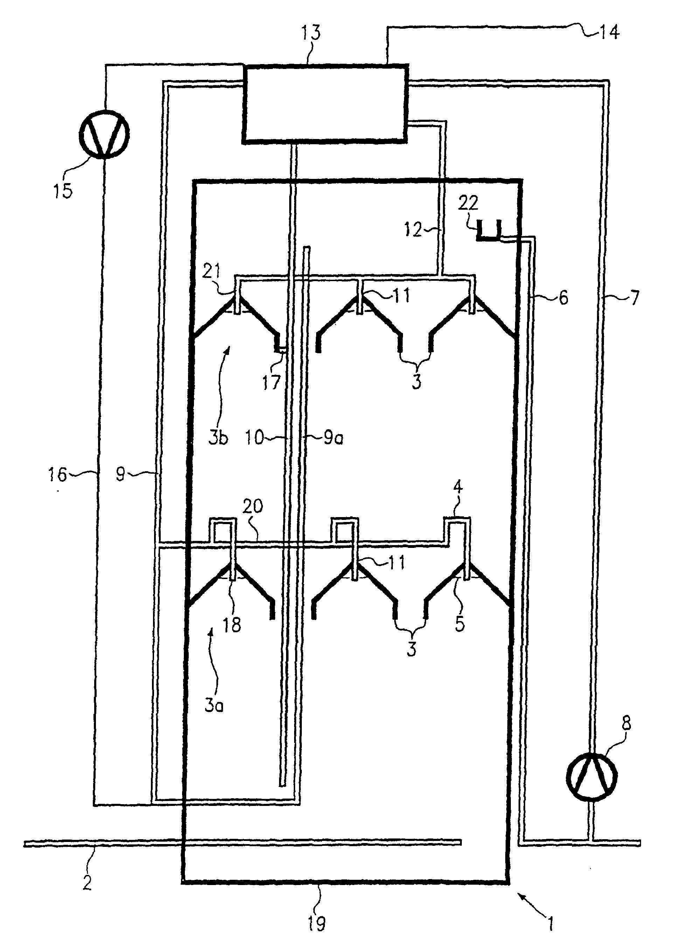

Ein Ausführungsbeispiel einer erfindungsgemäßen Vorrichtung zum Durchführen eines Ausführungsbeispiels des erfindungsgemäßen Verfahrens ist der beiliegenden einzigen Figur zu entnehmen.

In der Figur ist eine erfindungsgemäße Vorrichtung 1 zum anaeroben Reinigen von Abwasser nach dem UASB-Verfahren ersichtlich, die jedoch nur schematisch dargestellt ist. Die Vorrichtung ist eine Weiterentwicklkung der Vorrichtung, die in der nicht vorveröffentlichten deutschen Patentanmeldung 198 15 616 beschrieben ist, auf die hiermit Bezug genommen wird.

Die Vorrichtung 1 enthält einen aufrechtstehenden Behälter 19, in den im Bodenbereich, in dem sich eine mit Mikroorganismen versetzte Sinkschlammzone befindet, eine Abwasserleitung 2 einmündet. Die Abwasserleitung 2 kann mit einem derüblichen Verteilssysteme für das Abwasser versehen sein, das jedoch nicht dargestellt ist.

Über die Höhe des Behälters 19 gestaffelt sind eine Vielzahl von Gassammlem 3 angeordnet. Die Gassammler 3 sind im dargestellten Ausführungsbeispiel in zwei Systeme, d.h. einem unteren System 3a und einem oberen System 3b gruppiert. Jeder der Gassammler 3 ist in Form einer der bekannten Gassammelhauben ausgebildet, mit einer nach unten weisenden Öffnung, die sich über den größten horizontalen Querschnitt der Haube erstreckt und mit einem sich nach oben verjüngenden, vertikalen Querschnitt. Es können jedoch auch andere Formen von Gassammlern eingesetzt werden.

Die Gassammler 3 des unteren Systems 3a des erfindungsgemäßen Behälters 19 weisen Leitungen 18 auf, die sich durch ihren Dachfirst 11 erstrecken und gasdicht mit dem First 11 verschweißt sind. Jede der Leitungen 18 erstreckt sich vom Dachfirst 11 noch ein Stück, bevorzugt das 0,1- bis 0,4-fache der Höhe der Gassammelhaube, nach unten in die Gassammelhaube hinein und weist eine horizontale, gegebenenfalls mit Schlammabweisern versehene Mündung auf. Oberhalb des Dachfirstes 11 weisen die Leitungen 18 eine umgekehrt U-förmige Krümmung 4 auf, die mit einer Abführleitung 20 verbunden ist. Die Einmündung der umgekehrt U-förmigen Krümmung 4 in die Abführleitung 20, bzw. die Einmündung der Abführleitung 20 in eine als Steigleitung 9 ausgebildete Gassammelleitung, liegt einige Zentimeter, vorzugsweise zwischen 5 und 10 cm, oberhalb der Mündung der Leitung 18 unterhalb des Dachfirstes 11. Die Leitung 20, die mit allen Gassammlem 3 des Systems 3a verbunden ist, mündet in die Steigleitung 9, d.h. eine Rohrleitung mit einem gegenüber dem Querschnitt des Behälters 19 stark verkleinerten Querschnitt. Die Steigleitung 9 führt nach oben in eine oberhalb des Behälters 19 angeordnete Gas-Schlamm-Trenneinrichtung 13. Von dieser Gas-Schlamm-Trenneinrichtung 13 führt eine Sinkleitung 10 zurück in den Behälter 19 und mündet mit einer nach unten weisenden, horizontalen Ausmündung unterhalb des unteren Systems 3a und knapp oberhalb oder innerhalb einer sich im Behälter 19 ausbildenden Sinkschlammzone.

Das obere System 3b der Gassammelhauben 3 dient in üblicher Weise dem Zurückhalten von Schwimmschlamm, damit dieser nicht in das gereinigte Abwasser gerät. Die Gassammler 3 des oberen Systems 3b weisen ebenfalls nach oben über den Dachfirst 11 ausmündende Leitungen 21 auf, die jedoch ohne die U-förmige Krümmung 4 des Systems 3a in eine Abführleitung 12 münden, die wiederum in die Gas-Schlamm-Trenneinrichtung 13 führt. Unterhalb der Dachfirste 11 sind die Leitungen 21 wie die Leitungen 18 des Systems 3a ausgebildet.

Einige oder alle Gassammler 3 des oberen Systems 3b können über eine Rohrleitung 17 mit der Sinkleitung 10 verbunden sein. Die Rohrleitung 17 liegt in demjenigen Teil der Gassammler 3, in dem sich bevorzugt der Schwimmschlamm ansammelt.

Oberhalb des oberen Systems 3b befindet sich ein den oberen Wasserspiegel im Behälter 19 bestimmender Überlauf 22 für das gereinigte Wasser, der in eine Wasserleitung 6 mündet. Aus der Wasserleitung 6 führt eine Rückführleitung 7, die mit einer Pumpe 8 versehen ist und in die Gas-Schlamm-Trenneinrichtung 13 zurück. Aus der Gas-Schlamm-Trenneinrichtung 13 führt eine erste Gasleitung 14 heraus, die das gewonnene Biogas zur Weiterverarbeitung, beispielsweise für eine Energiegewinnung, wegführt. Aus der Gas-Schlamm-Trenneinrichtung 13 führt eine weitere Gasleitung 16 heraus, in die ebenfalls eine Pumpe 15 eingeschaltet ist. Die Gasleitung 16 führt in den unteren Bereichen der Steigleitung 9, so daß das Gas zur Unterstützung der Hebewirkung rückgeleitet werden kann.

Die Steigleitung 9 führt unterhalb der Abführleitung 20 mit einer Ansaugleitung 9a in den oberen Bereich des Behälters 19 und mündet dort unterhalb des Überlaufs 22 für das gereinigte Abwasser, d.h. knapp unterhalb des Wasserspiegels

Die erfindungsgemäße Vorrichtung arbeitet nach dem folgenden Verfahren. Über die Abwasserleitung 2 gelangt das zu reinigende Abwasser, beispielsweise organisch hoch belastetes Abwasser aus der Lebensmittel- oder Papierindustrie, in den unteren Bereich des Behälters 19 und in die dort vorhandene, mit Mikroorganismen versetzte Sinkschlammschicht, vorzugsweise in Form der üblichen Schlammpellets. Die Mikroorganismen bauen die organischen Belastungen des Abwassers ab, wodurch Gas entsteht. Das Gas perlt in Form von Gasblasen nach oben und bleibt auch den Schlammpellets hängen, so daß diese leichter werden und aufschwimmen. Das Gas und die als Schwimmschlamm aufschwimmenden, gasbeladenen Pellets gelangen in den Bereich des ersten Systems 3a und werden durch die Gassammler 3 aufgefangen. Das Gas sammelt sich in einem Gaspolster 5 unterhalb des Dachfirstes 11 und oberhalb einer Schicht aus Schwimmschlamm, der ebenfalls nach und nach seine Gasbeladung abgibt und als Sinkschlamm wieder zurücksinkt. Das Gas gelangt über die Leitung 18 in die U-förmige Krümmung 4. In dieser bildet sich ein Gaspolster, das das Aufsteigen und Mitreißen von Schwimmschlamm in die Leitung 20 verhindert. Dadurch gelangt allein Gas über die Leitung 20 in die Steigleitung 9. Dort verursacht das Gas eine starke, aufsteigende Strömung und dadurch einen Unterdruck an der Ansaugöffnung der Ansaugleitung 9a. Durch diesen Unterdruck wird Medium aus dem oberen Bereich des Behälters mit den noch vorhandenen Inhaltsstoffen, d.h. insbesondere bereits weitgehend gereinigtes Wasser mit Schwimmschlammresten, die vom Überlauf zurückgehalten wurden, bzw. eventuell Restgas, angesaugt und zusammen mit den mitgerissenen Partikeln und dem Gas aus den Gassammlern zurück in die Gas-Schlamm-Trenneinrichtung 13 geführt. Die Mischung aus Gas, angesaugtem Sinkschlamm und Wasser aus dem oberen Bereich des Behälters 19 sowie dem Bereich unterhalb der Gassammler wird in der Gas-Schlamm-Trenneinrichtung 13 getrennt, wobei das Gas normalerweise über die Leitung 14 zur Weiterverarbeitung geführt wird. Der mitgerissene Schlamm sammelt sich und wird durch das Wasser wieder nach unten über die Sinkleitung 10 in den Behälter 19 gespült, wo er unterhalb des Systems 3a in den Behälter entlassen wird, wiederauf den Boden sinkt und dort für Turbulenzen sorgt, so daß die Mikroorganismen immer von Abwasser umspült werden und somit optimale Lebensbedingungen vorfinden. Darüber hinaus wird das Abwasser einem zweiten Reinigungsprozeß unterzogen und es wird dafür gesorgt, daß sich am Überlauf 22 nicht so viel Restschlamm ansammelt, daß die Gefahr besteht, daß er in das gereinigte Wasser mitgerissen wird. Schließlich wird auf diese Weise ein vorteilhafter interner Reaktorumlauf erzeugt, ohne daß externe Energie, z.B. für eine Pumpe, zugeführt werden muß. Beim Absinken des Schlamms aus der Trenneinrichtung 13 durch die Sinkleitung 10 wird über das Rohr 17 Schwimmschlamm angesaugt, der sich unter den Gassammlern 3 des oberen Systems 3b angesammelt hat. Diese verstärkt den durch den zurückgeführten Schlamm bewirkten Verwirbelungseffekt. Eine weitere Verstärkung des Verwirbelungseffektes durch den zurücksinkenden Schlamm kann mit Hilfe einer kleinen Menge von bereits gereinigtem Abwasser erreicht werden, das durch die Pumpe 8 der Wasserleitung 6 entnommen und über die Leitung 7 wieder zurück in die Trenneinrichtung 13 gepumpt wird, wo es mithilft den Schlamm wirksam durch die Sinkleitung 10 auszuspülen.

Das Ansaugen kann, insbesondere beim Anlaufen der Vorrichtung 1, unterstützt werden, indem über die Leitung 16 und die Pumpe 15 Gas bzw. unter Umständen auch Luft in die Steigleitung 9 gepumpt wird.

Der Schwimmschlamm und die Gasblasen, die noch nicht vom unteren System 3a eingefangen wurden, bzw. dort bei Überschreitung der Sammelkapazität wieder ausgetreten sind, gelangen in den Bereich des oberen Systems 3b und werden dort von den Gassammlern 3 eingefangen. Das sich im First 11 sammelnde Gas wird über die geradlinigen, vertikalen Leitungen 21 und die Leitung 12 in die Trenneinrichtung 13 geleitet, während die Schwimmschlammschicht entweder über das Rohr 17 abgeleitet wird oder, nach Abgabe des Gases, wieder zurück auf den Boden sinkt. Das gereinigte Abwasser fließt in den Überlauf 22 und gelangt dort in die-Wasserleitung 6.

Bei Bedarf kann die erfindungsgemäße Vorrichtung auch zusätzlich anaerob betrieben werden, wobei in die Gas-Schlamm-Trenneinrichtung 13 Sauerstoff in einer Menge von 1 bis 3 Vol.-% hinzugegeben wird, um beispielsweise eine biologische Oxidation des im Gas vorhandenen Schwefels zu erreichen. Dabei unterbleibt eine Rückführung des Gases über die Pumpe 15 und die Leitung 16.

Üblicherweise haben anaerobe Abbauprozesse ein Optimum zwischen 25°C und 37°C. Durch die höheren Strömungen ist es möglich, die Reaktionstemperatur abzusenken. Die optimale Temperatur für das erfindungsgemäße Verfahren liegt zwischen 10 und 37°C. Dies bedeutet eine erhebliche Energieeinsparung, da ein Erwärmen des Abwassers nicht mehr notwendig ist.

Weiterhin kann bei hohen Reaktoren der oberen Reaktorraum mit einer höhere Dichte an Pelletschlamm versehen werden, wobei die Leistungsfähigkeit nochmals gesteigert wird. Weiterhin können insbesondere bei eiweiß- oder stärkehaltigen Abwässern, die sogenannte aufschwimmende Koagulate bilden, diese sicher wieder in den Bodenbereich geleitet werden.

In Abwandlung des beschriebenen und gezeichneten Ausführungsbeispieles können mehr als zwei Gassammelsysteme vorgesehen sein. Bei besonders geringen Reaktorhöhen kann die Pumpe zur Rückführung von Gas in die Steigleitung im Dauerbetrieb laufen. Die Steigleitung kann im Inneren des Behälters und die Sinkleitung außerhalb des Behälters verlaufen. Es ist auch möglich, den Sinkschlamm statt in die Trennvorrichtung über einen Abzweig aus dem Steigrohr direkt in den Behälter zu führen. Wenn sichergestellt ist, daß allein oder überwiegend Gas in die Steigleitung gelangt, können die Gassammler ohne die U-förmige Krümmung bzw. mit einer anderen Gas-Trenneinrichtung ausgestattet bzw. mit einer solchen, vor die Steigleitung eingeschalteten Gas-Trenneinrichtung verbunden sein. Schließlich muß das über die Ansaugleitung angesaugte Medium, insbesondere wenn es wenig oder kein Gas mehr enthält, nicht über die Trenneinrichtung geführt werden, sondern kann direkt in den Behälter gelangen.

## Patentansprüche

1. Verfahren zum Reinigen von Abwasser, inbesondere anaerobe Reinigung, in einem ein Medium aufnehmenden Behälter unter Gasentwicklung, wobei das sich entwickelnde Gas durch Gassammler eines ersten, unteren Systems (3a) mit vom oberen Bereich der Gassammler (3) nach oben wegführenden Leitungen (18) und eines zweiten, oberhalb des ersten Systems (3a) vorgesehenen Systems (3b) aufgefangen, vom Gas mitgerissene Inhaltsstoffe des Mediums abgetrennt und die abgetrennten Inhaltsstoffe unterhalb des Gassammlers wieder in das Medium eingeleitet werden, **dadurch gekennzeichnet, daß** das Gas über die nach oben wegführenden Leitungen (18) und eine Abführleitung (20) in eine Steigleitung (9) geleitet wird, die unterhalb der Abführleitung (20) mit einer Ansaugleitung (9a) in den oberen Bereich des Behälters (19) führt, wobei durch einen Gashebeeffekt des aufsteigenden Gases über die Ansaugleitung Medium aus dem oberen Bereich des Behälters oberhalb des oberen Systems (3b) angesaugt und zurück in den Behälter geleitet wird.

2. Verfahren nach Anspruch **1, dadurch gekennzeichnet, daß** das im oberen Bereich des Behälters angesaügte Medium zusammen mit den vom Gas getrennten Inhaltsstoffen in den unteren Bereich des Behälters zurückgeleitet wird.

3. Vorrichtung zum Reinigen von Abwasser, insbesondere durch eine anaerobe Reinigung, mit einem das Medium aufnehmenden Behälter (19), einem Abwasserzulauf (2), einem Ablauf (22) für gereinigtes Wasser, im Behälter (19) angeordneten Gassammlern (3) eines ersten, unteren Systems (3a) mit vom oberen Bereich der Gassammler (3) nach oben wegführenden Leitungen (18) und eines zweiten, oberhalb des ersten Systems (3a) vorgesehenen Systems (3b) **dadurch gekennzeichnet, daß** das Gas über die nach oben wegführenden Leitungen (18) und eine Abführleitung (20) in eine Steigleitung (9) geleitet wird, die unterhalb der Abführleitung (20) mit einer Ansaugleitung (9a) in den oberen Bereich des Behälters (19) führt, wobei oberhalb des oberen System (3b) im oberen Bereich des Behälters (19) die Mündung der Ansaugleitung (9a) angeordnet ist, wobei in die Ansaugleitung (9a) die Leitung (18) vom Gassammler (3) so einmündet, daß das vom Gassammler (3) kommende Gas als Gasheber zum Ansaugen des Mediums in die Ansaugleitung (9a) einsetzbar ist,

4. Vorrichtung nach Anspruch 3, **dadurch gekennzeichnet, daß** die Ansaugleitung (9a) zusammen mit der Leitung (18) in eine Gassammelleitung (9) mündet, die in eine Gas-Schlamm-Trenneinrichtung (13) führt, aus der eine Sinkleitung (10) zurück in den Behälter (19) unter den Gassammler (3) führt.

5. Vorrichtung nach Anspruch 4, **dadurch gekennzeichnet, daß** im Gassammler (3) eine Gas-Trenneinrichtung (4) vorgesehen ist.

6. Vorrichtung nach Anspruch 5, **dadurch gekennzeichnet, daß** die Gas-Trenneinrichtung durch eine im wesentlichen umgekehrt U-förmige Krümmung (4) der aus dem Gassammler (3) nach oben wegführenden Leitung (18) gebildet ist.

7. Vorrichtung nach einem der Ansprüche 4 bis 6, **dadurch gekennzeichnet, daß** in die Sinkleitung (10) eine Rohrleitung (17) aus dem unteren Bereich des Gassammlers (3) mündet.

8. Vorrichtung nach einem der Ansprüche 3 bis 7, **dadurch gekennzeichnet, daß** die nach oben wegführenden Leitungen (18) des ersten, unteren Systems (3a) mit einer U-förmigen Krümmung (4) versehen sind.

9. Vorrichtung nach Anspruch 7 und 8, **dadurch gekennzeichnet, daß** die Rohrleitung (17) zwischen Gassammlern (3) des zweiten Systems (3b) und der Sinkleitung (10) vorgesehen sind.

10. Vorrichtung nach einem der Ansprüche 3 bis 10, **dadurch gekennzeichnet, daß** in die Steigleitung (9) eine zusätzliche Gasleitung (16) einmündet.

## Claims

1. A method of purifying waste water, in particular an anaerobic purification, in a container receiving a medium under the development of gas, wherein the gas developing is caught by gas collectors of a first lower system (3a) with lines (18) upwardly leading out of the upper portion of the gas collectors and of a second system (3b) provided above the first system (3a), ingredients of the medium entrained by the gas are separated and the ingredients separated are passed back into the medium below the gas collector, **characterized in that** the gas is passed via the upwardly extending lines (18) and a discharge line (20) into a riser (9) which below the discharge line (20) leads with an intake line (9a) into the upper portion of the container (19), wherein caused by a gas lifting effect of the rising gas, medium is taken in via the intake line from the upper portion of the container above the upper system and is conducted back into the container.

2. A method as claimed in claim 1, **characterized in that** the medium taken in in the upper portion of the container is returned together with the ingredients separated from the gas into the lower portion of the container.

3. A device for purifying waste water, in particular by an anaerobic purification, comprising a container (19) receiving the medium, a waste water intake (2), an outtake (22) for purified water, gas collectors (3) of a first lower system (3a) with lines (18) upwardly leading out of the upper portion of the gas collectors and of a second system (3b) provided above the first system (3a) arranged in the container (19), **characterized in that** the gas is passed via the upwardly extending lines (18) and a discharge line (20) into a riser (9) which below the discharge line (20) leads with an intake line (9a) into the upper portion of the container (19), wherein the orifice of the intake line (9a) is arranged above the upper system (3b) in the upper portion of the container (19), the line (18) from the gas collector (3) opens into the intake line (9a) in such a manner that the gas coming from the gas collector (3) can be used as a gas lifter for taking in the medium into the intake line (9a).

4. A device as claimed in claim 3, **characterized in that** the intake line (9a) together with the line (18) opens into a gas collector line (9), which leads into a gas-sludge separation means (13) from which a sinking line (10) leads back into the container (19) below the gas collector (3).

5. A device as claimed in claim 4, **characterized in that** a gas separation means (4) is provided in the gas collector (3).

6. A device as claimed in claim 5, **characterized in that** the gas separation means is formed of a substantially inverted U-shaped curvature (4) of the line (18) upwardly leading out of the gas collector (3).

7. A device as claimed in one of claims 4 to 6, **characterized in that** a pipeline (17) from the lower portion of the gas collector (3) opens into the sinking line (10).

8. A device as claimed in one of claims 3 to 7, **characterized in that** the upwardly extending lines (18) of the first lower system (3a) are provided with a U-shaped curvature (4).

9. A device as claimed in claims 7 and 8, **characterized in that** the pipeline (17) is provided between the gas collectors (3) of the second system (3b) and the sinking line (10).

10. A device as claimed in one of claims 3 to 10, **characterized in that** an additional gas line (16) opens into the riser (9).

## Revendications

1. Procédé d'épuration d'eaux usées, en particulier épuration anaérobie, dans une cuve contenant un fluide, avec dégagement gazeux, dans lequel le gaz qui se dégage est capté via des collecteurs de gaz d'un premier système inférieur (3a) comportant des conduites (18) s'éloignant de la zone supérieure vers le haut à partir des collecteurs de gaz et d'un second système (3b) prévu au-dessus du premier système (3a), des substances constitutives du fluide entraînées par le gaz sont séparées et les substances constitutives séparées sont réintroduites dans le fluide, au-dessous du collecteur de gaz, **caractérisé en ce que** le gaz est conduit par l'intermédiaire des conduites (18) s'éloignant vers le haut à partir des collecteurs de gaz et une d'une conduite d'évacuation (20) dans une conduite ascendante (9) qui mène au-dessous de la conduite d'évacuation (20) avec une conduite d'aspiration (9a) dans la région supérieure de la cuve (19), du fluide étant aspiré, par un effet d'entraînement gazeux du gaz ascendant via la conduite d'aspiration, de la zone supérieure de la cuve au-dessus du système supérieur et réintroduit dans la cuve.

2. Procédé selon la revendication 1, **caractérisé en ce que** le fluide aspiré dans la zone supérieure de la cuve est réintroduit dans la zone inférieure de la cuve avec les substances constitutives séparées du gaz.

3. Dispositif d'épuration d'eaux usées, en particulier par une épuration anaérobie, présentant une cuve (19) contenant le fluide, une arrivée d'eaux usées (2), une évacuation (22) pour l'eau épurée, des collecteurs de gaz (3), disposés dans la cuve (19), d'un premier système inférieur (3a) comportant des conduites (18) s'éloignant de la zone supérieure vers le haut à partir des collecteurs de gaz et d'un second système (3b) prévu au-dessus du premier système (3a), **caractérisé en ce que** le gaz est conduit par l'intermédiaire des conduites (18) s'éloignant vers le haut à partir des collecteurs de gaz et une d'une conduite d'évacuation (20) dans une conduite ascendante (9) qui mène au-dessous de la conduite d'évacuation (20) avec une conduite d'aspiration (9a) dans la région supérieure de la cuve (19), l'embouchure de la conduite d'aspiration (9a) étant disposée dans la zone supérieure de la cuve (19) au-dessus du système supérieur (3b), la conduite (18) du collecteur de gaz (3) débouchant de manière telle dans la conduite d'aspiration (9a) que le gaz arrivant du collecteur de gaz (3) peut être utilisé en tant que gaz entraîneur pour aspirer le fluide dans la conduite d'aspiration (9a).

4. Dispositif selon la revendication 3, **caractérisé en ce que** la conduite d'aspiration (9a) débouche avec la conduite (18) dans une conduite collectrice de gaz (9), qui mène à un dispositif de séparation gaz/boues (13), duquel une conduite descendante (10) retourne vers la cuve (19) sous le collecteur de gaz (3).

5. Dispositif selon la revendication 4, **caractérisé en ce qu'**un dispositif séparateur de gaz (4) est prévu dans le collecteur de gaz (3).

6. Dispositif selon la revendication 5, **caractérisé en ce que** le dispositif séparateur de gaz est formé par un coude (4) essentiellement en forme de U inversé de la conduite (18) s'éloignant vers le haut hors du collecteur de gaz (3).

7. Dispositif selon l'une des revendications 4 à 6, **caractérisé en ce qu'**une tuyauterie (17) venant de la zone inférieure du collecteur de gaz (3) débouche dans la conduite descendante (10).

8. Dispositif selon l'une des revendications 3 à 7, **caractérisé en ce que** les conduites (18) du premier système inférieur (3a) s'éloignant vers le haut comportant un coude (4) en forme de U.

9. Dispositif selon les revendications 7 et 8, **caractérisé en ce que** la tuyauterie (17) est prévue entre des collecteurs de gaz (3) du second système (3b) et la conduite descendante (10).

10. Dispositif selon l'une des revendications 3 à 10, **caractérisé en ce qu'**une conduite de gaz supplémentaire (16) débouche dans la conduite ascendante (9).
